# EUROPÄISCHE PATENTSCHRIFT

(11) **EP 3 678 604 B1**
(45) Veröffentlichungstag und Bekanntmachung des Hinweises auf die Patenterteilung: **01.06.2022**
(21) Anmeldenummer: 18765849.7
(22) Anmeldetag: 04.09.2018
(51) Int. Cl.: A61F 2/66, A61F 2/76

(54) **PROTHESE FÜR EINE UNTERE EXTREMITÄT UND VERBINDUNGSEINRICHTUNG FÜR EINE SOLCHE**
PROSTHESIS FOR LOWER EXTREMITY AND CONNECTING DEVICE
PROTHESE POUR EXTREMITE INFERIEURE ET DISPOSITIF DE CONNEXION

(30) Priorität: 04.09.2017 DE 102017120257
(43) Veröffentlichungstag der Anmeldung: 15.07.2020
(73) Patentinhaber: Ottobock SE & Co. KGaA, 37115 Duderstadt (DE)
(72) Erfinder: KRENZ, Hannes, 01309 Dresden (DE)
(74) Vertreter: Gramm, Lins & Partner Patent- und Rechtsanwälte PartGmbB
(86) Internationale Anmeldenummer: PCT/EP2018/073749
(87) Internationale Veröffentlichungsnummer: WO 2019/043254

(56) Entgegenhaltungen:
- DE-A1-102015 225 438
- US-A- 5 425 763
- US-A- 5 507 837
- US-A1- 2011 030 174
- US-A1- 2012 143 350

## Beschreibung

Die Erfindung betrifft eine Verbindungseinrichtung für eine Prothese für eine untere Extremität, wobei die Prothese ein erstes Prothesenbauteil und ein zweites Prothesenbauteil aufweist, wobei das erste Prothesenbauteil relativ zu dem zweiten Prothesenbauteil durch wenigstens ein Verriegelungselement verriegelbar ist, wobei die Prothese wenigstens eine Halteeinrichtung aufweist, durch die das erste Prothesenbauteil an dem zweiten Prothesenbauteil lösbar gehalten wird, wie in den Ansprüchen 1-8 definiert. Die Erfindung betrifft zudem eine Prothese mit einer derartigen Verbindungseinrichtung, wie in Anspruch 9 definiert.

Derartige Prothesen sind beispielsweise aus der DE 10 2015 225 438 A1 bekannt. Ähnliche Prothesen sind der US 5,326,352 und der US 5,507,837 A zu entnehmen.

Für Prothesenträger einer Prothese für die untere Extremität, beispielsweise einer Beinprothese oder einer Fußprothese, ist es in unterschiedlichen Situationen sinnvoll, bestimmte Bauteile der Prothese auszutauschen und so beispielsweise einen Prothesenfuß, der für herkömmliches Gehen und Stehen geeignet ist, gegen einen Sportfuß auszutauschen, wenn sich der Patient sportlich betätigen will. In anderen Fällen kann es sinnvoll sein, ein oder mehrere Prothesenbauteile vollständig zu entfernen, die beispielsweise auf langen Autofahrten störend wirken und nicht verwendet. Es gibt daher seit langer Zeit im Stand der Technik Bemühungen, verschiedene Prothesenbauteile möglichst schnell und einfach voneinander lösen und wieder aneinander befestigen zu können. So ist beispielsweise aus der US 2003/0650647 ein Prothesenadapter bekannt, bei dem ein rohrförmiges Prothesenteil in eine Schelle eingeführt wird, deren Umfang oder Querschnitt durch eine Klemmvorrichtung vergrößert oder verkleinert werden kann. Zum Entfernen des rohrförmigen Prothesenteils wird die Schelle aufgeweitet und das Prothesenbauteil kann entfernt werden. Soll es wieder montiert werden, wird es in die aufgeweitete Schelle eingesetzt und über die Klemmvorrichtung, die beispielsweise ein Schnellspannhebel sein kann, der Querschnitt der Schelle verringert und das Prothesenbauteil so eingeklemmt. Nachteilig ist jedoch, dass eine exakte Positionierung insbesondere in axialer Richtung des rohrförmigen Prothesenelementes und eine Winkeleinstellung der beiden miteinander zu verbindenden Prothesenbauteile nicht reproduzierbar einstellbar sind. Die entsprechenden eingestellten Informationen gehen beim Entfernen der Prothesenbauteile voneinander verloren. Ein anderer Prothesenadapter ist der US 9693884 B1 zu entnehmen.

Die Lehre der US 5,326,352 bietet daher den Vorteil, dass die beiden Bauteile der Prothese nur in bestimmten Winkelorientierungen zueinander und nur in einem Abstand zueinander befestigt werden können. Dazu verfügt die Prothese über einen Vorsprung an einem Prothesenbauteil und eine entsprechende Vertiefung in dem anderen Prothesenbauteil. Werden diese Elemente so aneinander angeordnet, dass der Vorsprung in die Ausnehmung eingeführt ist, sind der relative Abstand der beiden Bauteile sowie ihre Winkelorientierung zueinander festgelegt. In diesem Fall sind in dem Vorsprung und in dem jeweils anderen Prothesenbauteil vorhandene Bohrungen in Überdeckung zueinander gebracht worden, so dass diese mit einem Stift, der in diesem Fall das Verriegelungselement bildet, zueinander verriegelt werden.

Die Verriegelung zweier Prothesenbauteile zueinander bedeutet, dass eine Bewegung der beiden Bauteile relativ zueinander nicht mehr möglich ist. Dabei sind Bewegungen, die aufgrund von Spiel zwischen den Prothesenbauteilen oder Fertigungstoleranzen entstehen, nicht gemeint.

Nachteilig ist jedoch, dass die beiden Bauteile beim Verriegeln in der jeweiligen Verriegelungsposition, in der eine Verriegelung möglich ist, festgehalten oder auf sonstige Weise festgestellt werden müssen. Dies kann beispielsweise dadurch geschehen, dass die Prothese belastet wird. Dies ist jedoch einerseits aufwendig und insbesondere für motorisch eingeschränkte Personen nicht immer einfach durchzuführen und kann zu einem Unsicherheitsgefühl beim Träger der Prothese führen. Der Erfindung liegt daher die Aufgabe zugrunde, eine Prothese gemäß dem Oberbegriff des Anspruchs 1 so weiterzuentwickeln, dass sie sicher und einfach verwendbar ist.

Die Erfindung löst die gestellte Aufgabe durch eine Prothese gemäß dem Oberbegriff des Anspruchs 1, die sich dadurch auszeichnet, dass die Halteeinrichtung ein erstes Halteelement, das an dem ersten Prothesenbauteil angeordnet ist und einen Vorsprung mit einer Bohrung aufweist, und ein zweites Halteelement aufweist, das an dem zweiten Prothesenbauteil angeordnet ist und einen Grundkörper mit einer Bohrung aufweist, wobei die Bohrungen so in Überdeckung bringbar sind, dass das Verriegelungselement in die Bohrungen einführbar ist.

Wird die erfindungsgemäße Prothese für die untere Extremität, beispielsweise eine Bein- oder Fußprothese, verwendet, sind die beiden Prothesenbauteile miteinander verbunden. Das Verriegelungselement hat die beiden Prothesenbauteile zueinander verriegelt, so dass eine Bewegung der beiden Bauteile voneinander weg nicht möglich ist. Das Verriegelungselement kann dabei beispielsweise ein Stift, ein Clips oder eine Schraube sein. Es kann als separates Bauteil oder einstückig mit dem ersten Prothesenbauteil oder dem zweiten Prothesenbauteil ausgebildet sein. Selbstverständlich sind auch andere Verriegelungselemente möglich. Soll nun das erste Prothesenbauteil vom zweiten Prothesenbauteil entfernt werden, beispielsweise weil ein künstlicher Fuß durch einen anderen Fuß ersetzt werden soll, wird zunächst das Verriegelungselement gelöst, beispielsweise entfernt, wie dies aus dem Stand der Technik bekannt ist. Die Verriegelung wird auf diese Weise gelöst. Durch die erfindungsgemäß vorhandene Halteeinrichtung werden die beiden Prothesenbauteile jedoch noch immer aneinander fest gehalten, so dass beispielsweise beim unvorsichtigen Lösen des Verriegelungselementes es nicht zu einem unbeabsichtigten Entfernen des zweiten Prothesenbauteils vom ersten Prothesenbauteil kommen kann. Es ist somit nicht möglich, dass beispielsweise ein Prothesenfuß nach dem Lösen des Verriegelungselementes vom Rest der Prothese abfällt. Da die Halteeinrichtung die beiden Prothesenbauteile lösbar aneinander hält, können die beiden Prothesenbauteile leicht voneinander entfernt werden, indem beispielsweise eine Haltekraft, die in einer bevorzugten Ausgestaltung von der Halteeinrichtung aufgebracht wird, überwunden wird.

Die durch die Halteeinrichtung aufgebrachte Kraft, die die beiden Prothesenbauteile auch im entriegelten Zustand aneinander hält, ist vorzugsweise so groß, dass die beiden Prothesenbauteile nicht voneinander abfallen, wenn sie der Schwerkraft ausgesetzt sind. Die Kraft muss nicht so groß sein, dass die Prothesenbauteile auch bei der Benutzung der Prothese, beispielsweise in einer Schwungphase eines Schrittes aneinander gehalten werden, wenn sie nicht verriegelt sind. Die durch die Halteeinrichtung aufgebrachte Kraft beträgt beispielsweise wenigstens 10 N, vorzugsweise wenigstens 15 N, besonders bevorzugt wenigstens 20 N. Sie ist damit beispielsweise ausreichend, einen Prothesenfuß mit einem herkömmlichen Schuh zu halten.

Zum Montieren zweier Prothesenbauteile einer erfindungsgemäßen Prothese werden die beiden Prothesenbauteile so aneinander angeordnet, dass sie durch die Halteeinrichtung bereits gehalten werden. In diesem Zustand können die beiden Bauteile beispielsweise nicht mehr durch die wirkende Schwerkraft voneinander getrennt werden, so dass ein Herabfallen eines angesetzten oder eingesetzten Prothesenbauteils vom Rest der Prothese sicher vermieden wird. In diesem bereits sicheren Zustand hat der Prothesenträger beide Hände frei, um das Verriegelungselement einzusetzen und die beiden Prothesenbauteile relativ zueinander zu verriegeln und auf diese Weise Betriebssicherheit auch für die Benutzung der Prothese herzustellen.

Erfindungsgemäß verfügt die Halteeinrichtung über ein erstes Halteelement, das an dem ersten Prothesenbauteil angeordnet ist, und ein zweites Halteelement, das an dem zweiten Prothesenbauteil angeordnet ist. Die beiden Halteelemente können auch als Teil einer separaten Verbindungseinrichtung vorliegen, so dass auch bestehende Prothesen nachgerüstet werden und die Vorteile der Erfindung verwirklichen können. Vorteilhafterweise sind das erste Halteelement und das zweite Halteelement zueinander korrespondierend ausgebildete Formschlusselemente, insbesondere Klettverschlusselemente. Die beiden Prothesenbauteile werden zur Montage so einander angelegt, dass in diesem Fall die Formschlusselemente miteinander in Eingriff gebracht werden und so einen Lösen der beiden Prothesenbauteile voneinander nur noch über die Überwindung der entsprechenden Haltekraft, beispielsweise bei Klettverschluss- oder Druckknopfelementen, oder durch Betätigen einer entsprechenden Entriegelungseinrichtung, beispielsweise durch Verschieben eines Schnappelementes, voneinander getrennt werden können. Das in Eingriff bringen und Lösen ist jedoch einfach, vorteilhafterweise mit nur einer Hand, möglich. Das in Eingriff bringen der unterschiedlichen Formschlusselemente miteinander ist auch für motorisch eingeschränkte Personen einfach und sicher möglich ist. Dadurch wird eine Haltekraft aufgebracht, die ein versehentliches Verlieren eines oder beider der Prothesenteile verhindert, die jedoch vorteilhafterweise nicht ausreichend ist, um eine Betriebssicherheit der Prothese herzustellen. Dies geschieht erst dadurch, dass in diesem Zustand, in dem die Halteeinrichtung bereits wirkt, die Formschlusselemente beispielsweise bereits miteinander in Eingriff sind, das Verriegelungselement verwendet wird und die beiden Prothesenbauteile miteinander verriegelt werden.

Alternativ oder zusätzlich dazu kann beispielsweise durch das erste Halteelement auch eine Haltekraft, insbesondere eine magnetische Haltekraft, auf das zweite Halteelement ausübbar sein. Dazu hat es sich als vorteilhaft herausgestellt, wenn das erste Halteelement und/oder das zweite Halteelement wenigstens einen, bevorzugt mehrere Permanentmagnete, aufweist. Weisen beide Halteelement jeweils wenigstens einen Permanentmagnet auf, müssen selbstverständlich die Orientierungen der Magnete so gewählt werden, dass im aneinander angeordneten Zustand der beiden Prothesenbauteile ungleichnamige Pole der beiden Permanentmagneten einander zugewandt angeordnet sind, so dass es zu einer anziehenden Wechselwirkung und damit zu einer Haltekraft zwischen den beiden Halteelementen kommt. In einer konstruktiv einfacheren Ausgestaltung ist nur das erste Halteelement oder das zweite Halteelement mit wenigstens einem, vorzugsweise mehreren Magneten ausgestattet. Das jeweils andere Halteelement weist dabei wenigstens ein magnetisierbares Element auf, so dass eine Haltekraft zwischen den beiden Halteelementen entstehen kann. Dies ist insbesondere dann von Vorteil, wenn das zu lösende Prothesenbauteil, beispielsweise ein Prothesenfuß, der vom Rest einer angelegten Beinprothese oder eine Beinprothese, die von einem angelegten Schaft entfernt werden soll, nicht mit einem Magneten ausgestattet ist. In diesem Fall kann verhindert werden, dass es zu unbeabsichtigten Anziehungskräften zwischen dem entfernten Prothesenbauteil und anderen, beispielsweise metallischen, Gegenständen kommt.

Vorteilhafterweise ist das erste Halteelement an einem ersten Kontaktelement und das zweite Halteelement an einem zweiten Kontaktelement angeordnet, die aneinander anliegen, wenn das erste Prothesenbauteil relativ zu dem zweiten Prothesenbauteil verriegelt ist. Dabei verfügt das erste Kontaktelement vorteilhafterweise über wenigstens einen Vorsprung und das zweite Kontaktelement über eine zu dem Vorsprung korrespondierend ausgebildete Ausnehmung. Dies hat den Vorteil, dass beispielsweise eine Orientierung der beiden Prothesenbauteile im einander angelegten und insbesondere im verriegelten Zustand, festgelegt und damit reproduzierbar einstellbar ist. Zudem können die Bauteile selbst orientierend zueinander ausgebildet sein, was, wie dem Fachmann aus dem Stand der Technik bekannt ist, durch bestimmte Formgebungen des Vorsprungs und/oder der Ausnehmung erreicht werden kann. Dadurch wird die Handhabbarkeit der Prothese und damit ihre Akzeptanz beim Patienten weiter erhöht.

Vorzugsweise ist die Halteeinrichtung einhändig und vorzugsweise ohne Werkzeug lösbar, wozu vorteilhafterweise eine Bewegung in nur einer Richtung notwendig ist. Eine solche Bewegung ist beispielsweise eine Bewegung, die einhändig durchgeführt werden kann, ohne "umzufassen", also ohne das jeweilige Prothesenbauteil loslassen und in einer anderen Konfiguration neu fassen zu müssen. Sie kann eine Zugbewegung in einer Richtung sein, die nicht zwangsläufig exakt geradlinig, sondern auch gebogen ausgeführt werden kann.

Vorteilhafterweise sind die Prothesenbauteile nur verriegelbar, wenn die Halteeinrichtung die Prothesenbauteile aneinander hält und/oder die Halteeinrichtung kann nur gelöst werden, wenn die Prothesenbauteile entriegelt sind. Damit wird insbesondere eine Reihenfolge festgelegt, in der die einzelnen Schritte durchgeführt werden müssen. Beim Verbinden der Prothesenbauteile wird zunächst die Halteeinrichtung betätigt, indem die Halteelemente aneinander angeordnet werden, so dass die Kraft ausgeübt wird. Dabei kommt es vorzugsweise bereits zu einer Positionierung der Prothesenbauteile zueinander derart, dass die Verriegelungseinrichtung mit dem Verriegelungselement betätigt und verriegelt werden kann. Beim Lösen der beiden Bauteile voneinander müssen die beiden Bauteile zunächst entriegelt werden, bevor die durch die Halteeinrichtung aufgebrachte Kraft überwunden und die beiden Prothesenbauteile voneinander getrennt werden können.

Die Erfindung löst die gestellte Aufgabe zudem durch eine Verbindungseinrichtung für eine Prothese, wobei die Verbindungseinrichtung eine Halteeinrichtung mit einem ersten Halteelement zum Anordnen an einem ersten Prothesenbauteil und einem zweiten Halteelement zum Anordnen an einem zweiten Prothesenbauteil sowie das Verriegelungselement aufweist. Die Halteeinrichtung ist eingerichtet, die entsprechende Haltekraft aufzubringen. Die beiden Halteelemente können beispielsweise über vorgefertigte Adapter, angeordnete Verbindungselemente oder auf sonstige Weise mit dem jeweiligen Prothesenbauteil verbunden werden, so dass auch herkömmliche Prothesenbauteile in den Genuss der Vorteile der vorliegenden Erfindung kommen können.

Wird eine solche Verbindungseinrichtung in die zu verbindenden Prothesenbauteile kann dadurch einerseits das Gewicht der Prothese vorzugsweise um mehrere 100 g pro verwendeter Verbindungseinrichtung reduziert werden. Andererseits wird der benötigte Bauraum und insbesondere die Bauhöhe beispielsweise um 40 mm bis 80 mm je nach Prothesenbauteil reduziert, indem die distalen und proximalen Anschlussstücke eingespart werden können. Davon profitieren insbesondere Patienten, die einen langen Amputationsstupf, beispielsweise eine langen Oberschenkelstumpf oder Unterschenkelstumpf aufweisen, bei denen herkömmliche Verbindungseinrichtungen aufgrund der benötigten Bauhöhe nicht verwendet werden können. Durch die Integration der Verbindungseinrichtung in die Bauteile wird dies geändert.

Durch die hier beschriebenen Prothesen und Verbindungseinrichtungen können unterschiedliche Prothesenbauteile schnell, einfach und sicher miteinander verbunden werden. So kann beispielsweise eine Alltagsprothese schnell gegen eine Sportprothese, beispielsweise eine Schwimmprothese ausgetauscht werden. Dies ist nicht auf einzelne Komponenten, beispielsweise künstliche Füße oder Knie beschränkt. Auch ganze Prothesenteile aus mehreren Bauteilen können ausgetauscht werden. So kann eine gesamte Oberschenkprothese ausgetauscht werden, wobei beispielsweise nur der Prothesenschaft am Amputationsstumpf verbleibt.

Mit Hilfe der beiliegenden Figuren wird nachfolgend ein Ausführungsbeispiel der vorliegenden Erfindung näher erläutert. Es zeigt:
- Figur 1: - eine Verbindungseinrichtung gemäß einem ersten Ausführungsbeispiel der vorliegenden Erfindung im nicht verbundenen Zustand,
- Figur 2: - die Verbindungseinrichtung aus Figur 1 im verbundenen Zustand,
- Figur 3: - die Verbindungseinrichtung in einer ersten Schnittdarstellung,
- Figur 4: - die Verbindungseinrichtung in einer zweiten Schnittdarstellung,
- Figuren 5 bis 11: - schematische Darstellungen zum Wechseln eines Prothesenfußes bei einer Prothese gemäß einem weiteren Ausführungsbeispiel der vorliegenden Erfindung und
- Figuren 12 bis 14: - in Prothesenbauteile integrierte Verbindungseinrichtungen.

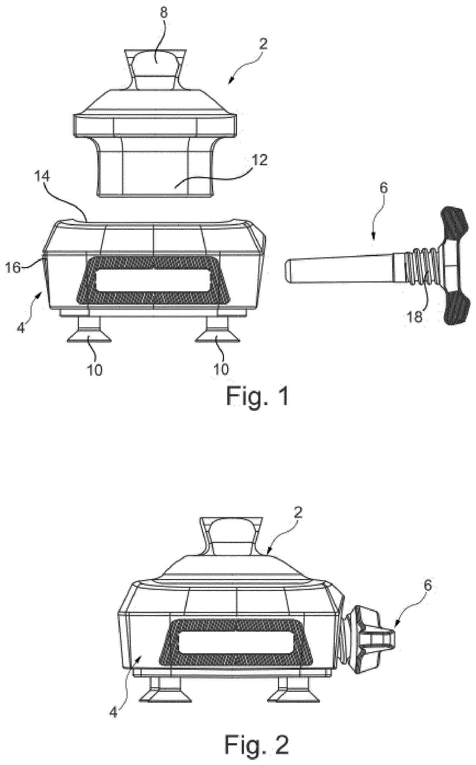

Figur 1 zeigt eine Verbindungseinrichtung gemäß einem ersten Ausführungsbeispiel der vorliegenden Erfindung. Sie verfügt über ein erstes Halteelement 2, ein zweites Halteelement 4 und ein Verriegelungselement 6. Das erste Halteelement 2 ist über ein Befestigungselement, das im gezeigten Ausführungsbeispiel ein Pyramidenadapter 8 ist, mit einem nicht dargestellten ersten Prothesenbauteil verbindbar. Am zweiten Halteelement 4 sind Schrauben 10 dargestellt, mit denen das zweite Halteelement 4 an einem ebenfalls nicht dargestellten zweiten Prothesenbauteil befestigbar ist. Das erste Halteelement 2 verfügt über einen Vorsprung 12, der in eine im zweiten Halteelement 4 vorhandene Ausnehmung 14 einsetzbar ist. In diesem Zustand sind Bohrungen, die sowohl in den Vorsprung 12 als auch in einem Grundkörper 16 des zweiten Halteelementes 4 vorhanden sind, so in Überdeckung gebracht, dass das Verriegelungselement 6 in die Bohrungen eingeführt und über ein Außengewinde 18, das mit einem in einer der Bohrungen vorhandenen Innengewinde korrespondierend ausgebildet ist, befestigbar ist.

Diese Situation ist in Figur 2 dargestellt. Man erkennt das erste Halteelement 2, das in das zweite Halteelement 4 eingesetzt ist. Zudem ist das Verriegelungselement 6 in die nicht gezeigten Bohrungen eingeschoben und über die Gewinde verschraubt worden.

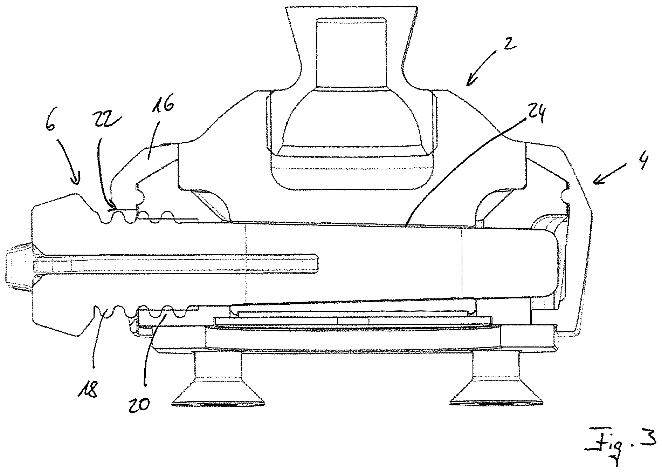

Figur 3 zeigt die Situation in einer Schnittdarstellung. Das Außengewinde 18 des Verriegelungselementes 6 greift in ein Innengewinde 20 in einer Bohrung 22 im Grundkörper 16 des zweiten Halteelementes 4 ein. Die Bohrung 22 ist im gezeigten Ausführungsbeispiel konisch ausgebildet. Dies ist zwar vorteilhaft, jedoch nicht zwingend notwendig. Im ersten Halteelement 2 befindet sich eine entsprechende Bohrung 24, die sich im gezeigten Ausführungsbeispiel vollständig durch das erste Halteelement 2 hindurch erstreckt. Das Verriegelungselement 6 ragt durch die beiden Bohrungen 22, 24 hindurch und verriegelt so die beiden Halteelemente 2, 4 und damit auch an diesen Halteelementen 2, 4 angeordnete, nicht dargestellte Prothesenbauteile miteinander.

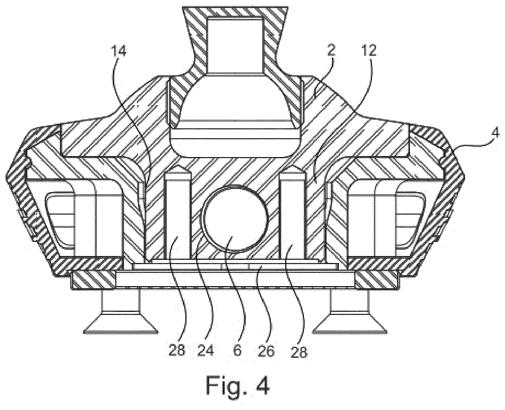

Figur 4 zeigt eine Schnittdarstellung durch die in Figur 2 gezeigte Situation um 90° gedreht relativ zum Schnitt in Figur 3. Man blinkt nun entlang der Längserstreckungsrichtung des Verriegelungselementes 6, das sich in der Bohrung 24 im ersten Halteelement 2 befindet.

Das zweite Halteelement 4 verfügt im gezeigten Ausführungsbeispiel über eine Metallplatte 26, die am Grund der Ausnehmung 14, in der sich der Vorsprung 12 des ersten Halteelementes 2 befindet, angeordnet sind. In diesem Vorsprung 12 sind im gezeigten Ausführungsbeispiel zwei Permanentmagnete 28 angeordnet, durch die eine Haltekraft auf die Metallplatte 26 ausgeübt wird. Selbstverständlich können anstelle der Metallplatte 26 auch weitere Permanentmagnete vorhanden sein. Auf diese Weise ist die Verbindungseinrichtung in der Lage, eine Haltekraft zwischen den beiden Halteelementen 2, 4 aufzubringen.

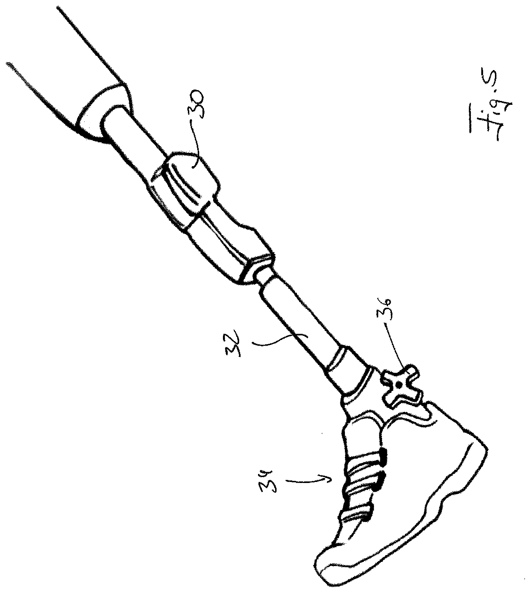

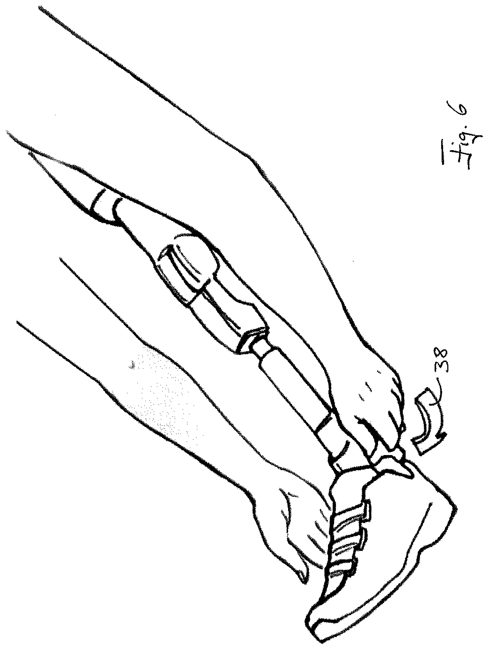

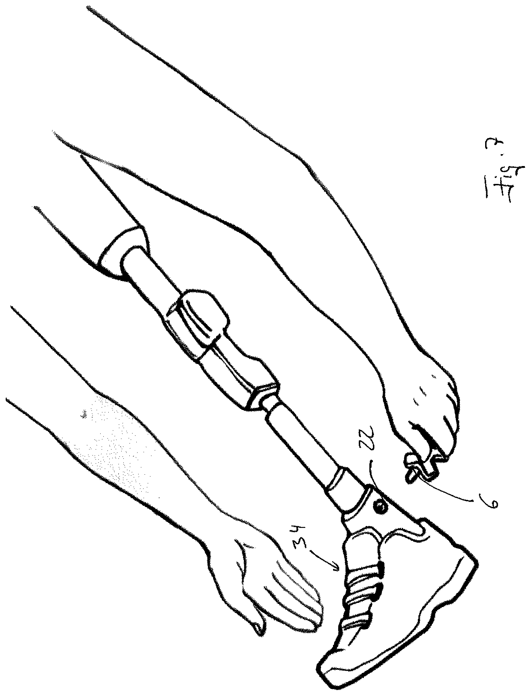

Figur 5 zeigt schematisch eine Prothese gemäß einem Ausführungsbeispiel der vorliegenden Erfindung. Sie verfügt über ein künstliches Knie 30 und einen daran befestigten künstlichen Unterschenkel 32, an dem ein sich in einem Schuh befindender Fuß 34 angeordnet ist. Eine andere Ausführungsform der vorliegenden Erfindung betrifft eine Unterschenkelprothese für Patienten, die noch über ein natürliches Kniegelenk verfügen. Die folgenden Ausführungen gelten in diesem Fall analog und sind auch auf andere Ausführungsbeispiele übertragbar. Zwischen dem Fuß 34 und dem künstlichen Unterschenkel 32 befindet sich die Verbindungseinrichtung, von der in der gezeigten Darstellung nur ein Betätigungselement 36 des Verriegelungselementes 6 dargestellt ist. Soll nun der Fuß 34 ausgetauscht werden, muss zunächst entlang der in Figur 6 dargestellten Pfeilrichtung 38 das Verriegelungselement 6 gelöst werden. Durch Drehen des Betätigungselementes 36 und damit des Verriegelungselementes 6 in Pfeilrichtung 38 wird das Außengewinde 18 mit dem Innengewinde 20 außer Eingriff gebracht. Es ist dann möglich, wie in Figur 7 dargestellt, das Verriegelungselement 6 aus der Bohrung 22 und der nicht dargestellten Bohrung 24 zu entfernen. Man erkennt, dass es nicht notwendig ist, den Fuß 34 festzuhalten, da durch die beiden Halteelemente 2, 4, die die Halteeinrichtung bilden, eine Haltekraft aufgebracht wird. Der Fuß 34 kann folglich nicht herunterfallen.

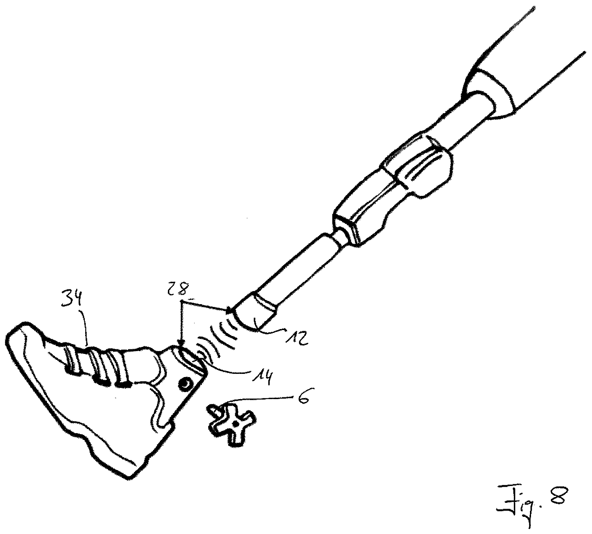

In Figur 8 ist der gelöste Zustand gezeigt. Im Vorsprung 12 des ersten Halteelementes 2 sowie in der Ausnehmung 14 des zweiten Halteelementes 4 sind jeweils Permanentmagnete 28 angeordnet, die aufeinander eine anziehende Kraft ausüben. Diese ist nur schematisch angedeutet. Das Verriegelungselement 6 ist weiterhin aus den Bohrungen 22, 24 entfernt.

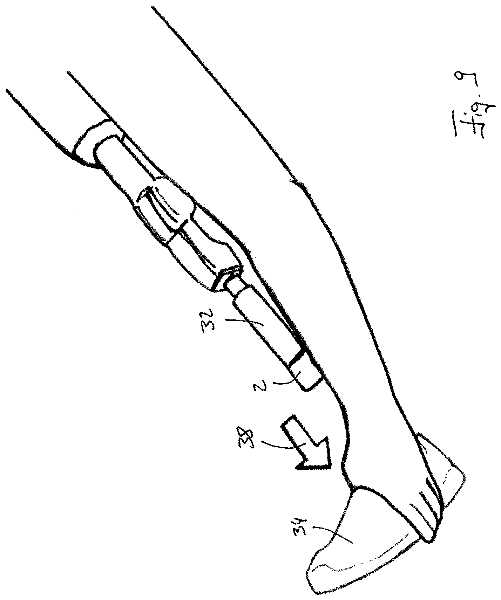

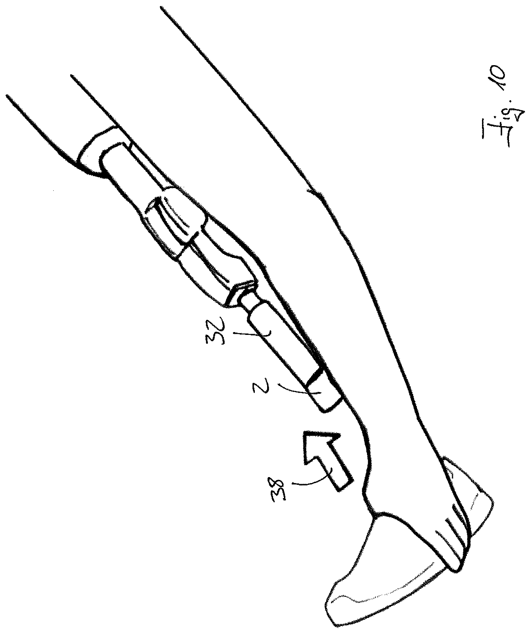

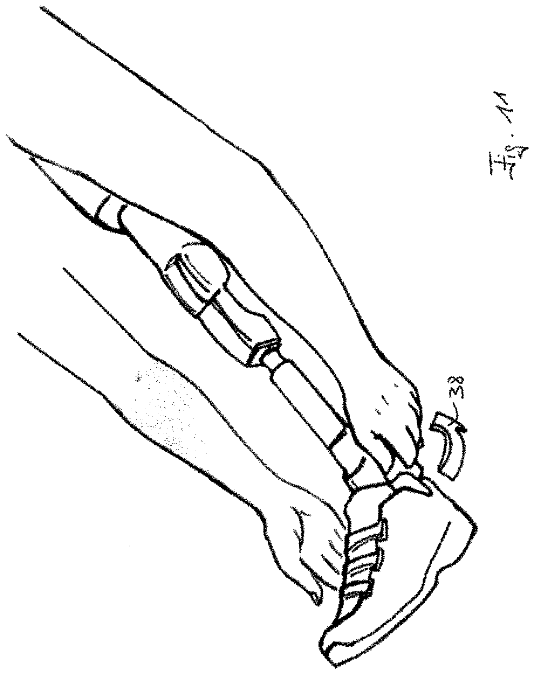

Der Fuß 34 ist nun vom Rest der Prothese entfernt und kann ausgetauscht werden. In Figur 9 ist bereits ein neuer Fuß 34 vorhanden, der nun montiert werden soll. Dazu wird das an diesem angeordnete nicht dargestellte zweite Halteelement 4 mit dem ersten Halteelement 2, das sich am künstlichen Unterschenkel 32 befindet, so in Eingriff gebracht, dass durch die beiden Halteelemente 2, 4, die die Halteeinrichtung bilden, eine Haltekraft ausgeübt wird. Dazu muss das erste Halteelement 2 entlang der Pfeilrichtung 38 in Figur 9 und das zweite Halteelement 4 entlang der Pfeilrichtung 38 in Figur 10 bewegt werden. Anschließend kann wie in Figur 11 dargestellt, das nicht gezeigte Verriegelungselement 6 in die Bohrungen 22, 24 eingeführt und entlang der Pfeilrichtung 38 in Figur 11 arretiert werden.

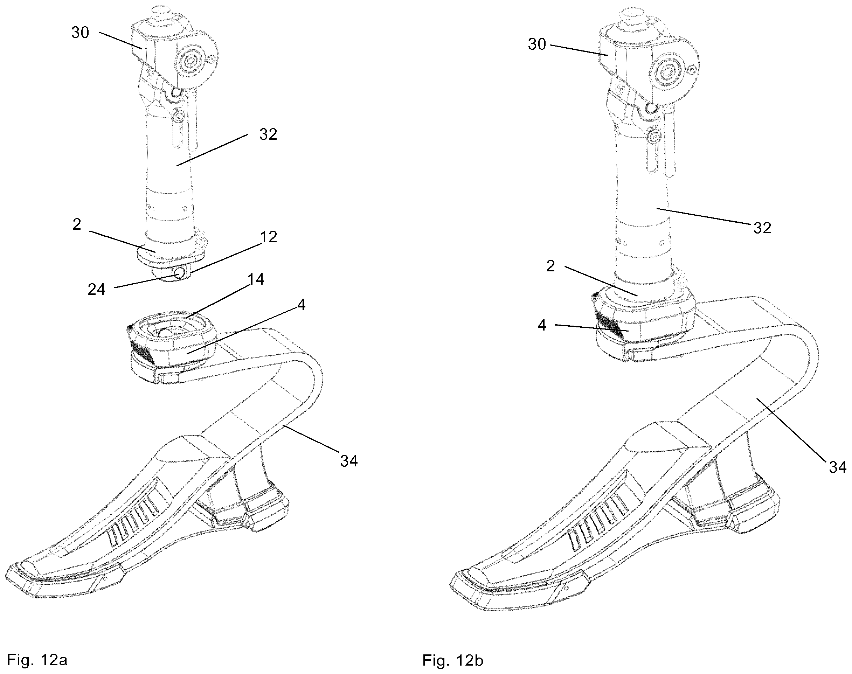

Die Figuren 12a und 12b zeigen als erstes Prothesenbauteil einen künstlichen Unterschenkel 32, an dessen oberen Ende ein künstliches Knie 30 angeordnet ist. Am unteren Ende ist das erste Halteelement 2 integriert, wobei in Figur 12a der Vorsprung 12 zu erkennen ist, in dem sich die Bohrung 24 für das Verriegelungselement 6, das in den Figuren 12a und 12b nicht gezeigt ist, befindet. Als zweites Prothesenbauteil ist ein künstlicher Fuß 34 dargestellt, an dessen oberen Ende das zweite Halteelement 4 integriert ist. In dessen Ausnehmung 14 wird der Vorsprung 12 des ersten Halteelementes 2 eingeführt. Figur 12b zeigt die Ausführung im miteinander verbundenen Zustand. Im Vergleich zu einer Ausführungsform, bei der ein herkömmlicher künstlicher Unterschenkel mit einem herkömmlichen künstlichen Fuß verbunden wird, wobei das erste Halteelement 2 und das zweite Halteelement 4 als separate Bauteile ausgebildet sind, die an den jeweiligen Prothesenbauteilen angeordnet sind, können auf diese Weise bis zu 80 mm Einbauhöhe und bis zu 300 g Gewicht der Prothese eingespart werden.

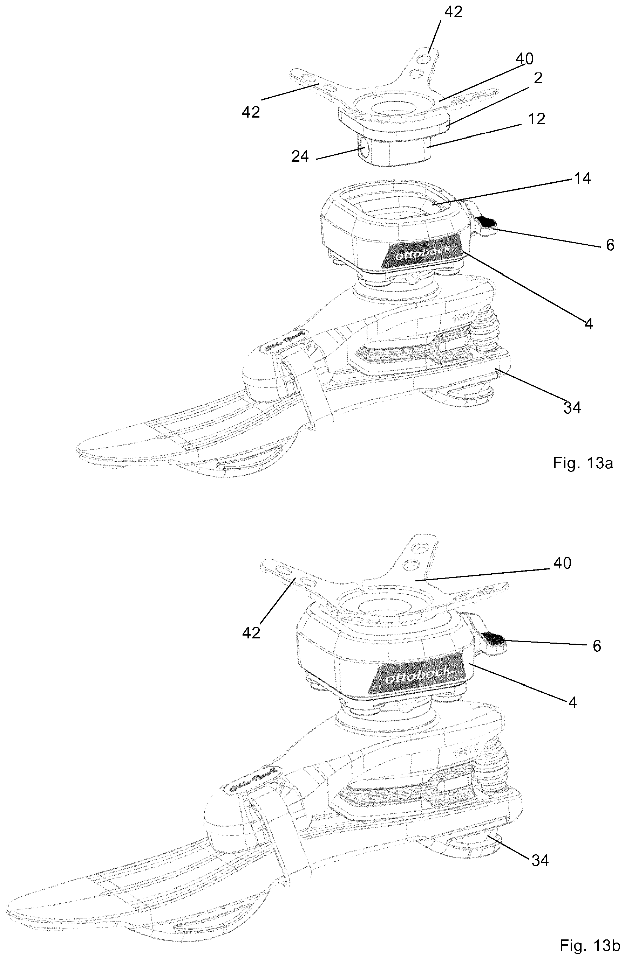

Figuren 13a und 13b zeigen zwei weitere Prothesenbauteile. Dargestellt ist der künstliche Fuß 34, der nun mit einem sogenannten Eingussadapter 40 verbunden werden soll. Ein Eingussadapter dient dazu, an einen noch herzustellenden und in den Figuren 13a und 13b nicht dargestellten Prothesenschaft weitere Bauteile anordnen zu können. Dieser Eingussadapter 40 verfügt über vier Arme 42, die beispielsweise in das Material des Prothesenschaftes eingegossen werden können. Eingussadapter 40 gibt es mit unterschiedlicher Anzahl von Armen 42. Auch Eingussadapter ohne Arme 42 werden verwendet. An der Unterseite des Eingussadapters 40 befindet sich wieder der Vorsprung 12 des ersten Halteelementes 2, in dem wieder die Bohrung 24 für das Verriegelungselement 6 dargestellt ist. Am künstlichen Fuß 34 befindet sich das zweite Halteelement 4 mit der Ausnehmung 14. Dargestellt ist zudem das Verriegelungselement 6, durch das die beiden Halteelemente 2, 4 miteinander verriegelt werden. Figur 13a zeigt den unverbundenen und Figur 13b den verbundenen Zustand.

Bei der in den Figuren 13a und 13b gezeigten Ausführungsform können bis zu 40 mm Einbauhöhe und bis zu 100 g Gewicht der Prothese eingespart werden. Bei der Verwendung in einem Kniegelenk können beispielsweise 40 mm Einbauhöhe und bis zu 200 g Gewicht eingespart werden.

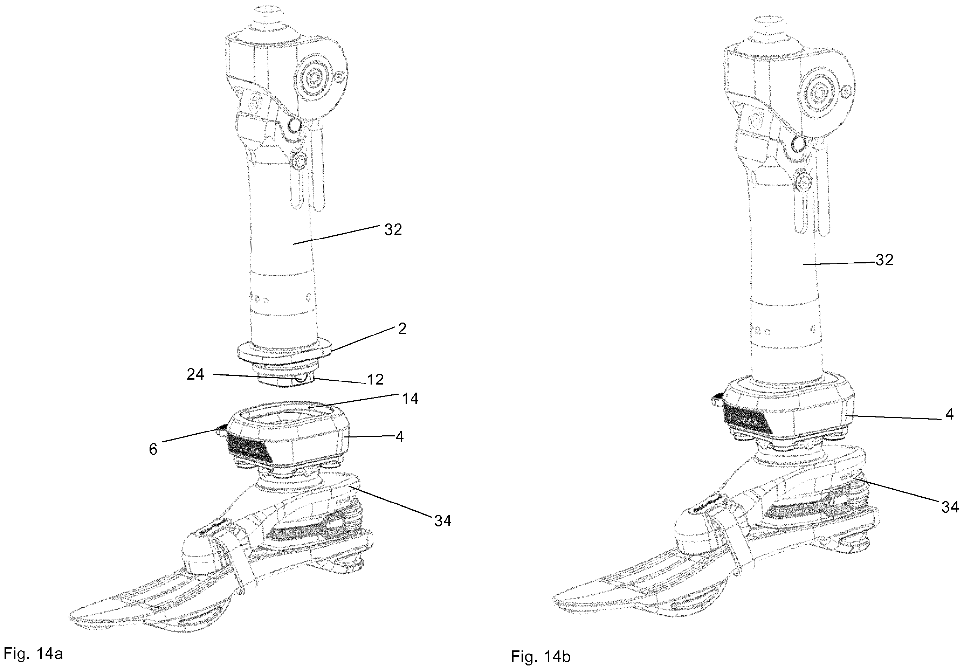

Die Figuren 14a und 14b zeigen eine weitere Ausführungsform. Die Darstellung entspricht den Darstellungen aus Figuren 12a und 12b. Am künstlichen Unterschenkel 32 ist das erste Halteelement 2 mit dem Vorsprung 12 angeordnet, während am künstlichen Fuß 34 das zweite Halteelement 4 mit der Ausnehmung 14 positioniert ist. Man erkennt zudem das Verriegelungselement 6, das im verriegelten Zustand durch die in Figur 14a dargestellte Bohrung 24 geführt wird. Durch derartige Ausgestaltungen kann eine große Einbauhöhe von beispielsweise 80 bis 90 mm eingespart werden, beispielsweise weil ein üblicher Doppeladapter mit einer Systemhöhe von allein 82 mm dann ersetzt werden kann.

### Bezugszeichenliste

- 2: erstes Halteelement
- 4: zweites Halteelement
- 6: Verriegelungselement
- 8: Pyramidenadapter
- 10: Schraube
- 12: Vorsprung
- 14: Ausnehmung
- 16: Grundkörper
- 18: Außengewinde
- 20: Innengewinde
- 22: Bohrung
- 24: Bohrung
- 26: Metallplatte
- 28: Permanentmagnet
- 30: künstliches Knie
- 32: künstlicher Unterschenkel
- 34: Fuß
- 36: Betätigungselement
- 38: Pfeilrichtung
- 40: Eingussadapter
- 42: Arm

## Patentansprüche

1. Verbindungseinrichtung für eine Prothese für eine untere Extremität umfassend ein erstes Prothesenbauteil und ein zweites Prothesenbauteil, wobei die Verbindungseinrichtung wenigstens ein Verriegelungselement (6) und eine Halteeinrichtung aufweist wobei das wenigstens eine Verriegelungselement gestaltet ist um das erste Prothesenbauteil relativ zu dem zweiten Prothesenbauteil zu verriegeln und wobei die Halteeinrichtung gestaltet ist um das erste Prothesenbauteil an dem zweiten Prothesenbauteil lösbar zu halten, wobei die Halteeinrichtung ein erstes Halteelement (2) und ein zweites Halteelement (4) aufweist, wobei das erste Halteelement an dem ersten Prothesenbauteil anordenbar ist und einen Vorsprung (12) aufweist, und das zweite Halteelement an dem zweiten Prothesenbauteil anordenbar ist und einen Grundkörper (16) mit einer Bohrung (22) aufweist, wobei der Vorsprung in eine im zweiten Halteelement vorhandene Ausnehmung (14) einsetzbar ist, **dadurch gekennzeichnet, dass** der Vorsprung des ersten Halteelements eine Bohrung (24) aufweist und die Bohrungen so in Überdeckung bringbar sind, dass das Verriegelungselement in die Bohrungen einführbar ist.

2. Verbindungseinrichtung nach Anspruch 1, **dadurch gekennzeichnet, dass** das erste Halteelement (2) und das zweite Halteelement (4) zueinander korrespondierend ausgebildete Formschlusselemente, insbesondere Klettverschlusselemente, sind.

3. Verbindungseinrichtung nach einem der Ansprüche 1 oder 2, **dadurch gekennzeichnet, dass** durch das erste Halteelement (2) eine Haltekraft, insbesondere eine magnetische Haltekraft auf das zweite Halteelement (4) ausübbar ist.

4. Verbindungseinrichtung nach Anspruch 3, **dadurch gekennzeichnet, dass** das erste Halteelement (2) und/oder das zweite Halteelement (4) wenigstens einen, bevorzugt mehrere Permanentmagnete (28) aufweist.

5. Verbindungseinrichtung nach einem der vorstehenden Ansprüche, **dadurch gekennzeichnet, dass** das erste Halteelement (2) an einem ersten Kontaktelement und das zweite Halteelement (4) an einem zweiten Kontaktelement angeordnet sind, die aneinander anliegen, wenn das erste Prothesenbauteil relativ zu dem zweiten Prothesenbauteil verriegelt ist.

6. Verbindungseinrichtung nach Anspruch 5, **dadurch gekennzeichnet, dass** das erste Kontaktelement den Vorsprung (12) und das zweite Kontaktelement die zu dem Vorsprung (12) korrespondierend ausgebildete Ausnehmung (14) aufweist.

7. Verbindungseinrichtung nach einem der vorstehenden Ansprüche, **dadurch gekennzeichnet, dass** die Halteeinrichtung einhändig und vorzugsweise ohne Werkzeug lösbar ist, wozu vorteilhafterweise eine Bewegung in nur einer Richtung notwendig ist.

8. Verbindungseinrichtung nach einem der vorstehenden Ansprüche, **dadurch gekennzeichnet, dass** die Prothesenbauteile nur verriegelbar sind, wenn die Halteeinrichtung die Prothesenbauteile aneinander hält und/oder die Halteeinrichtung nur gelöst werden kann, wenn die Prothesenbauteile entriegelt sind.

9. Prothese für eine untere Extremität, wobei die Prothese ein erstes Prothesenbauteil und ein zweites Prothesenbauteil aufweist, **dadurch gekennzeichnet, dass** die Prothese eine Verbindungseinrichtung nach einem der Ansprüche 1-8 aufweist.

## Claims

1. A connection device for a lower limb prosthesis, comprising a first prosthesis component and a second prosthesis component, wherein the connection device has at least one locking element (6) and one holding device, wherein the at least one locking element is configured to lock the first prosthesis component relative to the second prosthesis component and wherein the holding device is configured to releasably retain the first prosthesis component to the second prosthesis component, wherein the holding device has a first holding element (2) and a second holding element (4), wherein the first holding element (2) can be arranged on the first prosthesis component and features a projection (12), and the second holding element can be arranged on the second prosthesis component and provides a base body (16) with a bore (22), wherein the projection can be inserted in a recess (14) of the second holding element, **characterized in that** the projection of the first holding element provides a bore (24) and the bores can be moved so that they overlap with one another in such a way that the locking element can be inserted into the bores.

2. The connection device according to claim 1, **characterized by** the fact that the first holding element (2) and the second holding element (4) are positive-locking elements, especially Velcro elements, that are designed to correspond to one another.

3. The connection device according to claims 1 or 2, **characterized by** the fact that a holding force, especially a magnetic holding force, can be exerted by the first holding element (2) on the second holding element (4).

4. The connection device according to claim 3, **characterized by** the fact that the first holding element (2) and/or the second holding element (4) comprise at least one, but preferably several, permanent magnets (28).

5. The connection device according to one of the above claims, **characterized by** the fact that the first holding element (2) is arranged on a first contact element and the second holding element (4) on a second contact element which lie next to one another when the first prosthesis component is locked relative to the second prosthesis component.

6. The connection device according to claim 5, **characterized by** the fact that the first contact element features the projection (12) and the second contact element features the recess (14) that is designed to correspond to the projection (12).

7. The connection device according to one of the above claims, **characterized by** the fact that the holding device can be released with one hand and preferably without a tool, which advantageously requires a movement in just one direction.

8. The connection device according to one of the above claims, **characterized by** the fact that the prosthesis components can only be locked when the holding device is holding the prosthesis components together and/or the holding device can only be released when the prosthesis components are unlocked.

9. A prosthesis for a lower extremity, wherein the prosthesis comprises a first prosthesis component and a second prosthesis component, **characterized by** the fact that the prosthesis comprises a connection device according to claims 1 to 8.

## Revendications

1. Dispositif de liaison pour une prothèse destinée à un membre inférieur, comprenant un premier composant de prothèse et un deuxième composant de prothèse, le dispositif de liaison comprenant au moins un élément de verrouillage (6) et un dispositif de retenue, ledit au moins un élément de verrouillage étant conçu pour verrouiller le premier composant de prothèse par rapport au deuxième composant de prothèse, et le dispositif de retenue étant conçu pour retenir le premier composant de prothèse de façon amovible sur le deuxième composant de prothèse, le dispositif de retenue comprenant un premier élément de retenue (2) et un deuxième élément de retenue (4), le premier élément de retenue pouvant être disposé sur le premier composant de prothèse et présentant une saillie (12), et le deuxième élément de retenue pouvant être disposé sur le deuxième composant de prothèse et présentant un corps de base (16) pourvu d'un perçage (22), la saillie pouvant être insérée dans un évidement (14) prévu dans le deuxième élément de retenue,
**caractérisé en ce que**
la saillie du premier élément de retenue présente un perçage (24), et les perçages peuvent être mis en coïncidence de telle sorte que l'élément de verrouillage peut être introduit dans les perçages.

2. Dispositif de liaison selon la revendication 1,
**caractérisé en ce que** le premier élément de retenue (2) et le deuxième élément de retenue (4) sont des éléments en coopération de forme, en particulier des éléments à fermeture velcro, réalisés de manière à se correspondre.

3. Dispositif de liaison selon l'une des revendications 1 ou 2,
**caractérisé en ce qu'**une force de retenue, en particulier une force de retenue magnétique, peut être exercée sur le deuxième élément de retenue (4) par le premier élément de retenue (2).

4. Dispositif de liaison selon la revendication 3,
**caractérisé en ce que** le premier élément de retenue (2) et/ou le deuxième élément de retenue (4) présente au moins un, de préférence plusieurs aimants permanents (28).

5. Dispositif de liaison selon l'une des revendications précédentes, **caractérisé en ce que** le premier élément de retenue (2) est disposé sur un premier élément de contact, et le deuxième élément de retenue (4) est disposé sur un deuxième élément de contact, qui sont en appui l'un contre l'autre lorsque le premier composant de prothèse est verrouillé par rapport au deuxième composant de prothèse.

6. Dispositif de liaison selon la revendication 5,
**caractérisé en ce que** le premier élément de contact comprend la saillie (12), et le deuxième élément de contact comprend l'évidement (14) réalisé de manière à correspondre à la saillie (12).

7. Dispositif de liaison selon l'une des revendications précédentes, **caractérisé en ce que** le dispositif de retenue peut être détaché d'une seule main et de préférence sans outil, ce qui nécessite avantageusement un mouvement dans une seule direction.

8. Dispositif de liaison selon l'une des revendications précédentes, **caractérisé en ce que** les composants de prothèse ne peuvent être verrouillés que lorsque le dispositif de retenue maintient les composants de prothèse l'un contre l'autre, et/ou le dispositif de retenue ne peut être détaché que lorsque les composants de prothèse sont déverrouillés.

9. Prothèse destinée à un membre inférieur, la prothèse comprenant un premier composant de prothèse et un deuxième composant de prothèse, **caractérisée en ce que** la prothèse comprend un dispositif de liaison selon l'une des revendications 1 à 8.
